Europäisches Patentamt

(19) European Patent Office

Office européen des brevets

(11) Publication number: **0 106 622**
**B1**

(12) # EUROPEAN PATENT SPECIFICATION

(45) Date of publication of patent specification: **15.07.87**

(51) Int. Cl.⁴: **A 61 L 2/04, C 02 F 1/02**

(21) Application number: **83306021.3**

(22) Date of filing: **05.10.83**

(54) Decontaminating distilled water withdrawing means.

(30) Priority: **07.10.82 FI 823405**

(43) Date of publication of application:
**25.04.84 Bulletin 84/17**

(45) Publication of the grant of the patent:
**15.07.87 Bulletin 87/29**

(84) Designated Contracting States:
**CH DE FR GB IT LI NL SE**

(56) References cited:
**DE-A-2 460 067**
**DE-A-2 541 601**
**GB-A- 829 272**
**GB-A-1 504 334**
**US-A-3 069 736**

(73) Proprietor: **Oy Santasalo-Sohlberg AB**
**Hankasuontie 46**
**SF-00390 Helsinki 39 (FI)**

(72) Inventor: **Makela, Paavo**
**Vyokatu 2 A**
**00160 Helsinki 16 (FI)**
Inventor: **Santasalo, Lauri**
**Kuusisaarenpolku 7**
**00340 Helsinki 34 (FI)**

(74) Representative: **Lee, Philip Graham et al**
**MARKS & CLERK 57/60 Lincoln's Inn Fields**
**London WC2A 3LS (GB)**

Courier Press, Leamington Spa, England.

EP 0 106 622 B1

## Description

This invention relates to apparatus for withdrawing distilled water comprising a distilled water withdrawal tube communicating with a pipeline along which the distilled water flows and provided with a valve means.

There has been disclosed a distilled water storage tank which is based on so-called hot storage. In a storage tank of this type no substantial impairment of distillate quality takes place, not even during prolonged storage.

There has also been disclosed a distilled water distribution system by means of which distilled water is conducted from a storage tank by a distribution system in the form of a pipeline, to various points of use. In this prior art distribution system the pipeline was constructed as a bead-like pipeline having no completely horizontal portions whatsoever, not any abrupt bends. Each withdrawal valve is located in the pipeline at withdrawal points where the distilled water is at a lowest point in the pipeline. In a distribution system of this kind, the quality of the distilled water remains good, owing to the circulation and to the higher temperature, through a long enough period, even behind each valve although a given valve might not be opened over a prolonged period.

Other relevant prior proposals, of which the applicant has been made aware, are those represented in U.S.A. Specification 3,069,736, British Specification 829,272 and West German Specifications 2,460,067 and 2,541,601 all of which relate to methods and apparatus for distillation or sterilisation of water or aqueous solutions.

At present, the problem is encountered in the withdrawing of distilled water that the distilled water cannot be withdrawn in an adequately sterile condition. In prior art designs, the withdrawal tube leads from the distillate tank, and the lower part of the withdrawal tube has a swivelling protective cup.

The withdrawal tube is naturally also fitted with a sanitation valve. A design like this fails to guarantee withdrawal of distilled water in sterile condition, even when the distilled water is withdrawn hot, because the ambient air invariably contains microbes which cause the distilled water to become potentially non-sterile.

It is desired in certain applications to withdraw the distilled water at a temperature significantly lower than about +40°C. Prior art designs are not applicable for such applications because the distilled water withdrawal will be non-sterile.

The object of the invention is to provide apparatus for withdrawing distilled water which allows sterile withdrawal of distilled water at a lower temperature.

The aim of the invention is attained by apparatus which is characterised firstly in that it further comprises a heating member which is disposed to heat at least the lower outlet part of the withdrawal tube so that the temperature of the lower outlet part at least is always higher than the desired minimum temperature, and secondly a liquid container through which the withdrawal tube is disposed to pass, said container containing cold liquid for the purpose of withdrawing the distilled water at a lower temperature.

The other characteristic features of the withdrawing means of the invention are stated in the appended claims 2—4.

It is to be understood that in the design taught by the invention the withdrawal tube, or at least its lower part, should always be kept about a given minimum temperature, such as e.g. +90°C, so that the microbes in the air are destroyed and the water is always withdrawn in sterile condition. The heating of the withdrawal tube is advantageously carried out by electric heating.

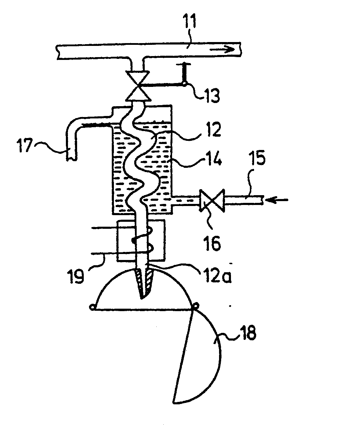

The invention is described in detail with reference to the attached drawing, but to which the invention is not meant to be exclusively confined.

The drawing shows apparatus for withdrawing distilled water according to the invention, intended for so-called cold withdrawal, in schematic elevational view.

In the embodiment of the drawing the distilled water flows continuously in the pipeline 11 and the temperature of the distilled water is at least a given minimum temperature, such as +80°C for instance. From the pipeline 11 there extends a distilled water withdrawal tube 12, with a lower part 12a and a sanitation valve 13. The withdrawal tube 12 passes through a liquid container 14 into which for example cold water is fed through a pipe 15. The temperature of the cold water may be e.g. +15°C. The pipe 15 has a valve 16 and the liquid container has a drain pipe 17. The lower part 12a of the withdrawal tube 12 has a swivelling protective cup of a kind known in the art. The embodiment depicted in the drawing is intended for so-called cold withdrawal, where it is desired to withdraw the water at a temperature for instance of about +40°C.

In accordance with the invention, at least the lower part 12a of the withdrawal tube 12 is heated so that the temperature of this lower part 12a is always over a given minimum temperature $T_{min}$, such as +90°C for instance. In the present embodiment, the heating is by means of an electric heater element 19. Due to this design, the microbes in the air are destroyed and the distilled water keeps reliably sterile, even when withdrawn cold.

## Claims

1. Apparatus for withdrawing distilled water, comprising a distilled water withdrawal tube (12) communicating with a pipeline (11) along which the distilled water flows and having a valve means (13), characterized in that the apparatus further comprises a heating member (19) which is disposed to heat at least the lower outlet part (12a) of the withdrawal tube (12) so that the temperature of the lower outlet part at least is over the desired minimum temperature ($T_{min}$) at all times, and a liquid container (14) through which the withdrawal tube (12) is disposed to pass, said container containing cold liquid for the purpose of withdrawing the distilled water at a lower temperature.

2. Withdrawing apparatus according to claim 1, characterized in that the heating member (19) is an electric heater element.

3. Withdrawing apparatus according to claim 1 or claim 2, characterized in that the minimum temperature ($T_{min}$) is about +90°C when the temperature of the distilled water flowing in the pipeline (11) is about +80°C.

4. Withdrawing apparatus according to any of claims 1—4, characterized in that the lower part (12a) of the withdrawal tube (12) has a swivelling protective cup (18) known *per se* in the art.

## Patentansprüche

1. Vorrichtung zum Entnehmen von destilliertem Wasser, mit einer Entnahmeröhre (12) für das destillierte Wasser, die mit einer Leitung (11), in der das destillierte Wasser fließt, in Verbindung steht, und einer Ventilvorrichtung (13), dadurch gekennzeichnet, daß die Vorrichtung desweiteren ein Heizelement (19), das zum Erwärmen zumindest des unteren Auslaßabschnittes (12a) der Entnahmeröhre (12) angeordnet ist, so daß die Temperatur des unteren Auslaßabschnittes jederzeit zumindest über einer gewünschten Minimal-Temperatur ($T_{min}$) liegt, und einen Flüssigkeitsbehälter (14) aufweist, durch den hindurch die Entnahmeröhre (12) verläuft, wobei der Flüssigkeitsbehälter eine kalte Flüssigkeit enthält, um das destillierte Wasser bei einer niedrigeren Temperatur zu entnehmen.

2. Entnahmevorrichtung nach Anspruch 1, dadurch gekennzeichnet, daß das Heizelement (19) ein elektrisches Heizelement ist.

3. Entnahmevorrichtung nach Anspruch 1 oder 2, dadurch gekennzeichnet, daß die Minimal-Temperatur ($T_{min}$) etwa +90°C beträgt, wenn die Temperatur des destillierten Wassers, das in der Leitung (11) fließt, etwa +80°C beträgt.

4. Entnahmevorrichtung nach einem der Ansprüche 1 bis 3, dadurch gekennzeichnet, daß der untere Abschnitt (12a) der Entnahmeröhre (12) eine an sich bekannte schwenkbare Schutzkappe (18) aufweist.

## Revendications

1. Appareil destiné au prélèvement d'eau distillée, comprenant un tube de prélèvement (12) d'eau distillée communiquant avec une conduite d'adduction (11) dans laquelle s'écoule l'eau distillée et comportant une vanne (13), caractérisé en ce qu'il comprend un élément chauffant (19) apte à chauffer au moins la partie de sortie inférieure (12a) du tube de prélèvement (12) de sorte que la température de la partie de sortie inférieure au moins se situe à tout moment au-dessus de la température minimale souhaitée ($T_{min}$) et un conteneur de liquide (14) traversé par une tube de prélèvement (12), ledit conteneur contenant le liquide froid pour le prélèvement de l'eau distillée à une température plus basse.

2. Appareil de prélèvement selon la revendication 1, caractérisé en ce que l'élément chauffant (19) est électrique.

3. Appareil de prélèvement selon la revendication 1 ou 2, caractérisé en ce que la température minimale ($T_{min}$) est d'environ +90°C lorsque la température de l'eau distillée s'écoulant dans la canalisation d'adduction (11) est d'environ +80°C.

4. Appareil de prélèvement selon l'une quelconque des revendications 1 à 3, caractérisé en ce que la partie inférieure (12a) du tube de prélèvement (12) comporte une coupelle protectrice pivotante (18) connue *per se*.